# NEW EUROPEAN PATENT SPECIFICATION

(11) **EP 2 313 087 B2**
(45) Date of publication and mention of the opposition decision: **22.11.2023**
(45) Mention of the grant of the patent: 21.11.2018
(21) Application number: 09757601.1
(22) Date of filing: 04.06.2009
(51) Int. Cl.: A61K 9/48, A61K 9/10, A61K 47/14, A61K 47/24, A61K 47/44, A61K 31/404

(54) **PHARMACEUTICAL DOSAGE FORM FOR IMMEDIATE RELEASE OF AN INDOLINONE DERIVATIVE**
PHARMAZEUTISCHE DOSIERUNGSFORM ZUR SCHNELLEN FREISETZUNG EINES INDOLINONDERIVATS
FORME POSOLOGIQUE PHARMACEUTIQUE POUR LIBÉRATION IMMÉDIATE DE DÉRIVÉ D'INDOLINONE

(30) Priority: 06.06.2008 EP 08157750
(43) Date of publication of application: 27.04.2011
(73) Proprietor: Boehringer Ingelheim International GmbH, 55216 Ingelheim am Rhein (DE)
(72) Inventor: MESSERSCHMID, Roman, 55216 Ingelheim Am Rhein (DE); LACH, Peter, 55216 Ingelheim Am Rhein (DE); SOKOLIESS, Torsten, 55216 Ingelheim Am Rhein (DE); STOPFER, Peter, 55216 Ingelheim Am Rhein (DE); TROMMESHAUSER, Dirk, 55216 Ingelheim Am Rhein (DE)
(74) Representative: Hoffmann Eitle
(86) International application number: PCT/EP2009/056895
(87) International publication number: WO 2009/147220

(56) References cited:
- WO-A-2004/013099
- WO-A-2004/096224
- WO-A-2006/018182
- WO-A-2006/067165
- WO-A-2007/054551
- WO-A1-2009/147212
- WO-A1-2009/147218
- WO-A1-2010/081817
- WO-A1-2010/103058
- WO-A1-2010/130757

## Description

The present invention relates to a pharmaceutical dosage form delivering an immediate release profile containing the active substance 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate.

### Background to the invention

The rate and extent to which the active ingredient or active moiety is absorbed from a pharmaceutical dosage form and becomes available at the site of action is defined as bioavailability (Chen, M. L. et al. , Bioavailability and bioequivalence: an FDA regulatory overview, Pharm. Res. 2001, 18, 1645-1648).

However, it is rarely feasible to measure the drug at the site of action. Therefore, bioavailability is assessed based on drug concentrations in the general circulation. The systemic exposure is determined by measuring the blood or plasma concentrations of the active drug at numerous time points following the drug administration and calculation of the area under the concentration- time curve (AUC). Blood/plasma drug concentration time profiles are affected by the dynamics of dissolution, solubility, absorption, metabolism, distribution, and elimination.

Drug absorption from a solid dosage form after administration depends on the release of the drug substance from the drug product, the dissolution or solubilization of the drug under physiological conditions, beside its permeability across the gut wall of the gastrointestinal tract. A higher dissolution rate of a formulation generally increases liberation out of the dosage form up to a maximum extent, which is a prerequisite for adequate bioavailability of an ingredient or active moiety. Because of the critical nature of this step, *in vitro* dissolution may be relevant to the prediction of *in vivo* plasma concentrations and therefore bioavailability. (Guidance for Industry, Dissolution Testing of Immediate Release Solid Oral Dosage Forms, U.S. Department of Health and Human Services, Food and Drug Administration, Center for Drug Evaluation and Research (CDER), August 1997). An observed *in vivo* difference in the rate and extent of absorption of a drug depends on the speed of drug dissolution *in vivo.* (Amidon, G. L. et al., A Theoretical Basis For a Biopharmaceutics Drug Classification: The Correlation of In Vitro Drug Product Dissolution and In Vivo Bioavailability, Pharmaceutical Research, 12: 413-420 (1995)).

Based on this general consideration, *in vitro* dissolution tests for immediate release solid oral dosage forms, such as tablets and capsules, are used to assess the quality of a drug product. An immediate release product allows the active to dissolve in the gastrointestinal tract, without causing any delay or prolongation of the dissolution or absorption of the drug. Requirements for dissolution testing of immediate release products are focused in the Guidance for Industry (CDER 1997) Dissolution testing for immediate release solid oral dosage forms, (CDER 1997) Immediate release solid oral dosage forms -Scale up and Postapproval Changes, ICH Guidance Q6A, Specifications: Test Procedures and Acceptance Criteria For New Drug Substances And New Drug Products. The most commonly employed dissolution test methods as described in the European Pharmacopeia 6.2 (6th edition) are the basket method (Apparatus 1) and the paddle method (Apparatus 2). The described methods are simple, robust, well standardized, and used worldwide. They are flexible enough to allow dissolution testing for a variety of drug products. Consistent with established regulatory guidance (e.g. European Pharmacopeia 6.2, 6th edition), the following parameters influencing the dissolution behaviour may for example be relevant for selecting the appropriate *in vitro* dissolution test conditions for an immediate release solid oral product: Apparatus, stirring speed, dissolution medium and temperature.

3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate is an innovative substance having valuable pharmacological properties, especially for the treatment of oncological diseases, immunologic diseases or pathological conditions involving an immunologic component, or fibrotic diseases.

The chemical structure of this substance is depicted below as Formula (I).

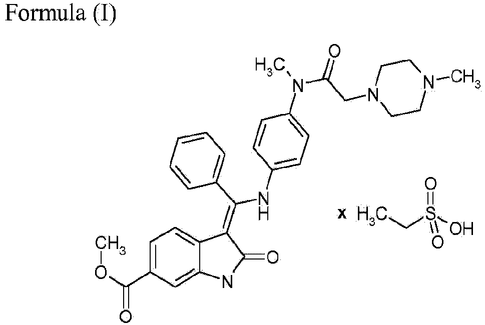

This substance is described as base in WO 01/27081, as monoethanesulfonate salt form in WO 2004/013099, for its use in the treatment of immunologic diseases or pathological conditions involving an immunologic component in WO 2004/017948 , for its use in the treatment of oncological diseases in WO 2004/096224, for its use in the treatment of fibrotic diseases in WO 2006/067165, and as other salt forms in WO 2007/141283.

The aim of the present invention is to obtain for the above drug substance a pharmaceutical dosage form which meets adequate bioavailability requirements for the desired target dosage range and which is further characterized by a specific immediate release profile range providing an appropriate plasma concentration-time profile of the active principle. Such specific release profile characteristic is not known from the prior art for this drug substance.

WO 2007/054551 discloses in a general context that pharmaceutical compositions suitable for two different therapeutic agents 1 and 2, the latter comprising 3-Z-[1-(4-(N-((4-methyl-piperazin-1-yl)-methylcarbonyl)-N-methyl-amino)-anilino)-1-phenylmethylene]-6-methoxycarbonyl-2-indolinone-monoethanesulphonate among many other options, may be in the form of an aqueous suspension or of oil-in-water emulsions which may inter alia comprise lecithin, without specifying the content of this component and without providing information regarding dissolution properties.

### Summary of the invention

A first object of the present invention is a pharmaceutical dosage form of the active substance 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate which delivers an immediate release profile in which not less than 70% (Q65%) of the active substance is dissolved in 60 minutes *in vitro* under the following *in vitro* dissolution conditions according to European Pharmacopeia 6.2: Apparatus 2 (paddle), dissolution medium with 0.1 M HCl (pH 1) and stirring speed of 50 to 150 rpm, at a temperature of 37°C, which comprises a viscous lipid suspension formulation of 10 to 50 weight % of the active substance in 10 to 70 weight % of medium chain triglycerides, 1 to 30% weight % of hard fat and 0.1 to 10 weight % of lecithin, based on the total weight of the viscous lipid suspension formulation.

In particular embodiments of the present invention the above pharmaceutical dosage form, under the above conditions, delivers an immediate release profile in which not less than 75% (Q 70%) of the active substance is dissolved in 60 minutes *in vitro.*

In particular embodiments of the present invention the above pharmaceutical dosage form, under the above conditions, delivers an immediate release profile in which not less than 85% (Q 80%) of the active substance is dissolved in 60 minutes *in vitro,* preferably not less than 85% (Q 80%) of the active substance is dissolved in 45 minutes *in vitro,* most preferably not less than 85% (Q 80%) of the active substance is dissolved in 30 minutes *in vitro.*

The above pharmaceutical dosage form, under the above conditions, exhibits comparable *in vitro* dissolution profiles independent from a dosage strength of 5 to 1000 mg of the active substance, preferably between 25 to 300 mg of the active substance.

A further object of the present invention is the above pharmaceutical dosage form, wherein it is an orally deliverable dosage form.

The lipid suspension of the above pharmaceutical dosage form according to the invention is dispersed in small droplets under the following *in vitro* dissolution conditions according to European Pharmacopeia 6.2: Apparatus 2 (paddle), dissolution medium with 0.1 M HCl (pH 1) and stirring speed of 50 to 150 rpm, at a temperature of 37° C.

A further object of the present invention is the above pharmaceutical dosage form in the form of a capsule, characterised in that the capsule shell is fast disintegrating *in vitro,* which is a prerequisite for fast liberation of the active *in vivo* as well, and characterized in that the capsule formulation is the above suspension of the active substance.

The pharmaceutical dosage form according to the invention is suitable for use as medicament, e.g. for use as pharmaceutical composition with an antiproliferative activity, for the treatment of a disease or condition selected from oncological diseases, immunologic diseases or pathological conditions involving an immunologic component, and fibrotic diseases.

The pharmaceutical dosage form according to the invention is suitable for the preparation of a medicament for the treatment of a disease or condition selected from oncological diseases, immunologic diseases or pathological conditions involving an immunologic component, and fibrotic diseases.

For the treatment and/or prevention of a disease or condition selected from oncological diseases, immunologic diseases or pathological conditions involving an immunologic component, and fibrotic diseases, an effective amount of the above defined pharmaceutical dosage form is administered orally to a patient once or several times daily.

A further object of the present invention is the above pharmaceutical dosage form which comprises dose-range values of between 25 to 300 mg of the active substance.

The above pharmaceutical dosage form may be used in a body-weight-independent (BWI) dosing.

Furthermore, the above pharmaceutical dosage form may be used in a dosage range of from 0.1 mg to 20 mg of active substance/kg body weight, preferably 0.5 mg to 5 mg active substance /kg body weight.

### Legend to the Figures

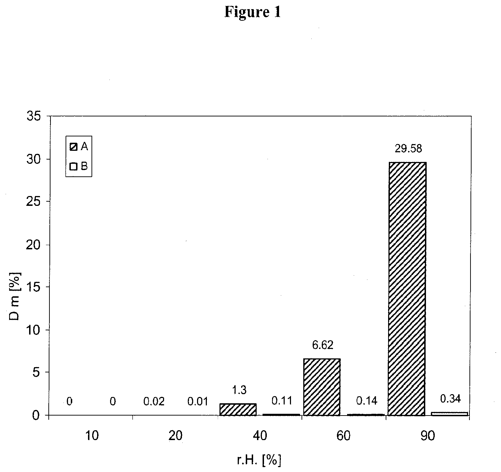

**Figure 1** **-** Mass gain by moisture sorption (Dm in %) under different relative humidity conditions (r.H. in %) for a soft gelatin capsule (A) and for a lipid suspension formulation (B).

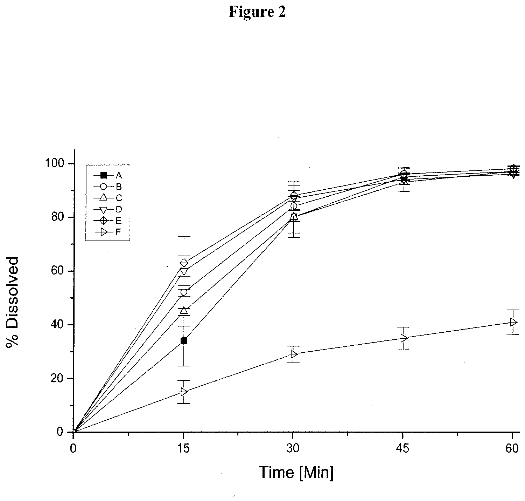

**Figure 2** - Effect of the employed lecithin amount in a 150 mg soft gelatin capsule on the *in vitro* dissolution behaviour. Dissolution tests with Apparatus 2 (paddle), 100 rpm, 900 mL pH 1.0 (0.1 M HCl) dissolution medium, 37°C: (A) 30% lecithin of preferred amount, (B) 75% lecithin of preferred amount, (C) 90% lecithin of preferred amount, (D) preferred amount of lecithin (equals to 100%), (E) 200% lecithin of preferred amount, (F) 0% lecithin.

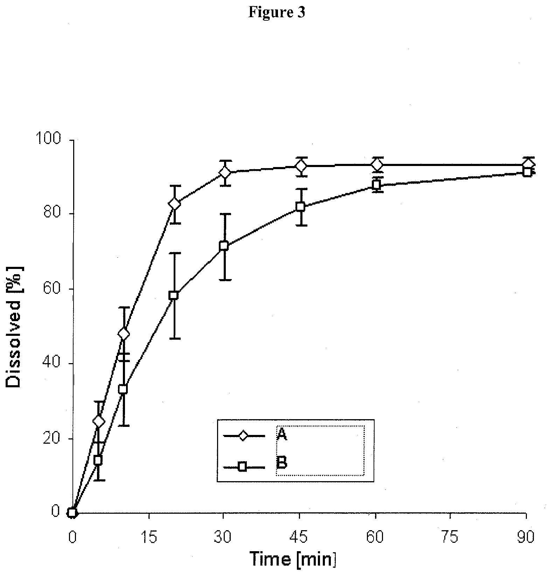

**Figure 3** **-** Effect of the melting range of the hard fat on the *in-vitro* dissolution behaviour (in % of dissolution) over time (in minutes) of soft gelatin capsules. Dissolution tests with Apparatus 2 (paddle), 100 rpm, 900 mL pH 1.2 dissolution medium, 37°C: (A) melting range of 33°C - 40°C, (B) melting range of 40°C - 44 °C.

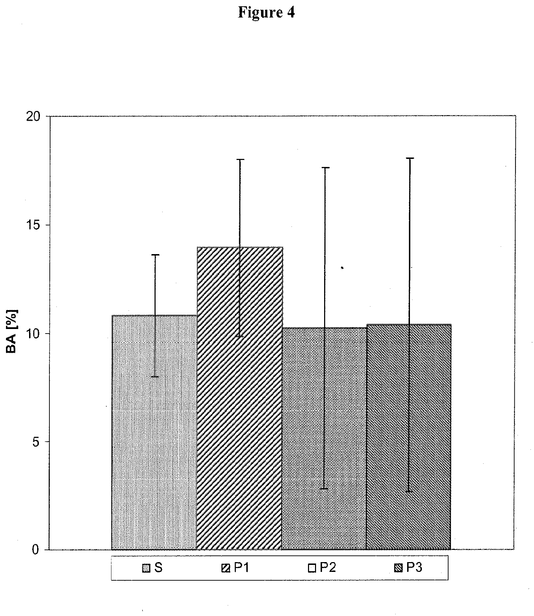

**Figure 4** - Comparison of the absolute bioavailability (BA in %) tested in the rat over 24 hours for the aqueous solution (S) versus different carrier systems (P1, P2 and P3) of the active substance - Error bars indicate standard deviations.

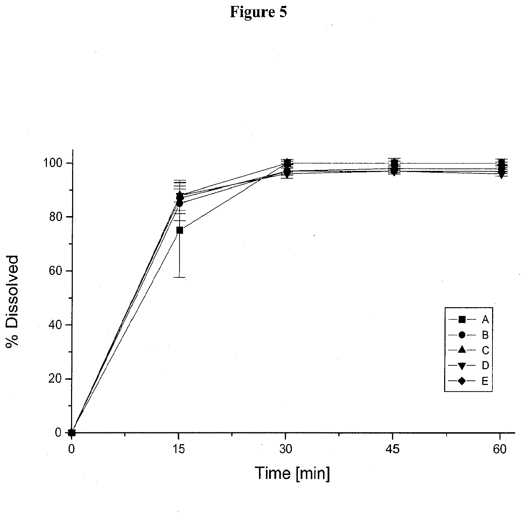

**Figure 5** - Influence of the dosage strength in the range 50 mg -150 mg of active substance (compound 3-Z-[1 -(4-(N-((4-methyl-piperazin-1-yl)-methylcarbonyl)-N-methyl-amino)-anilino)-1-phenyl-methylene]-6-methoxycarbonyl-2-indolinone-monoethanesulphonate) on the *in vitro* dissolution behaviour of soft gelatin capsules. Dissolution tests with Apparatus 2 (paddle), 100 rpm, 900 mL pH 1.0 (0.1 M HCl) dissolution medium, 37°C: (A) 50 mg active substance, (B) 75 mg active substance, (C) 100 mg active substance, (D) 125 mg active substance, (E) 150 mg active substance.

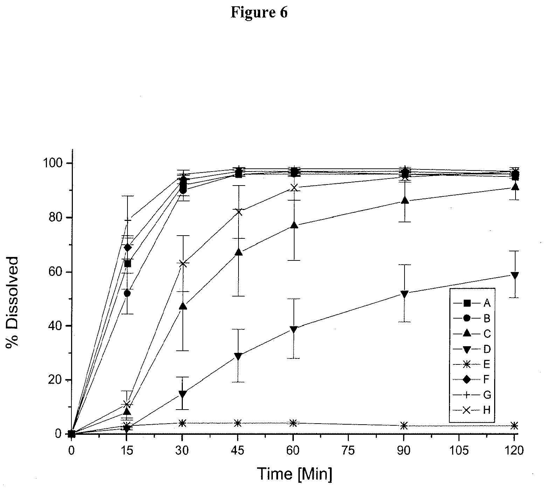

**Figure 6** **-** Influence of the dissolution media pH and the presence of surfactants on the *in vitro* dissolution behaviour of 150 mg soft gelatin capsules. Dissolution profile comparison of 150 mg soft gelatin capsules in the dissolution media pH 1.0 and pH 3.0, with and without surfactants (reference). Dissolution tests with Apparatus 2 (paddle), 100 rpm, 900 mL dissolution media in the pH range 1.0 to 6.8, 37°C: (A) pH 1.0, (B) pH 2.0, (C) pH 3.0, (D) pH 4.0, (E) pH 6.8, (F) pH 1.0 and 0.5% Cremophor, (G) pH 1.0 and 0.5% Tween 80, (H) pH 3.0 and 0.5% Tween 80.

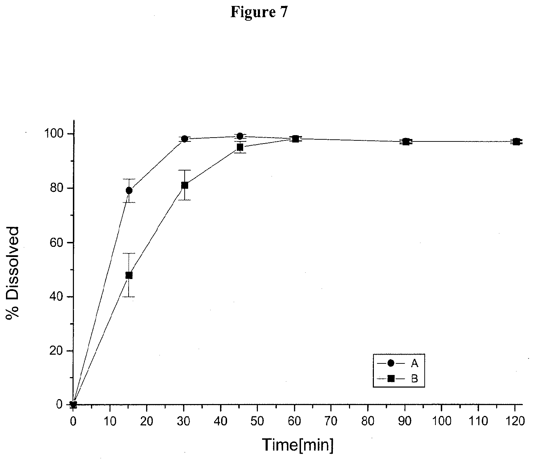

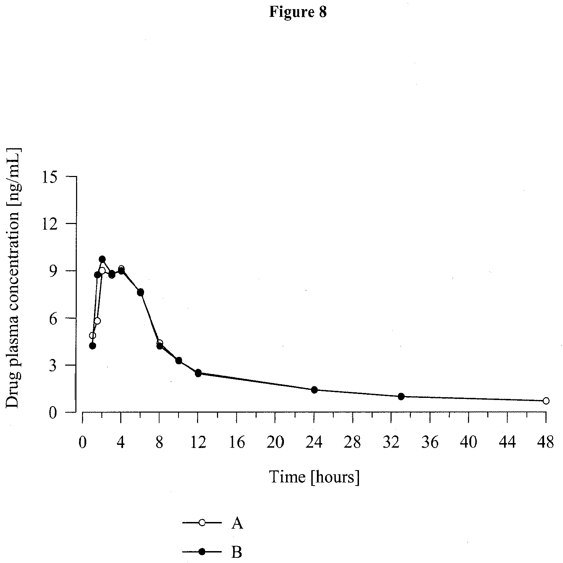

**Figure 7** - Dissolution curve of different 50 mg soft gelatin capsule batches used in the study of Figure 8, showing a fast and a slow *in vitro* dissolution profile. Dissolution tests with Apparatus 2 (paddle), 100 rpm, 900 mL pH 1.0 (0.1 M HCl) dissolution medium, 37°C: (A) fast, (B) slow.
**Figure 8** **-** Geometric mean plasma concentration - time profiles of formulations of the compound 3-Z-[1-(4-(N-((4-methyl-piperazin-1-yl)-methylcarbonyl)-N-methyl-amino)-anilino)-1-phenyl-methylene]-6-methoxycarbonyl-2-in-dolinone-monoethanesulphonate with slower (A, 3 x 50 mg soft gelatin capsules) and with faster in vitro release (B, 3 x 50 mg soft gelatin capsules) in a human bioavailability study. The plasma concentration refers to the compound 3-Z-[1-(4-(N-((4-methyl-piperazin-1-yl)-methylcarbonyl)-N-methyl-amino)-anilino)-1-phenyl-methylene]-6-methox-ycarbonyl-2-indolinone.

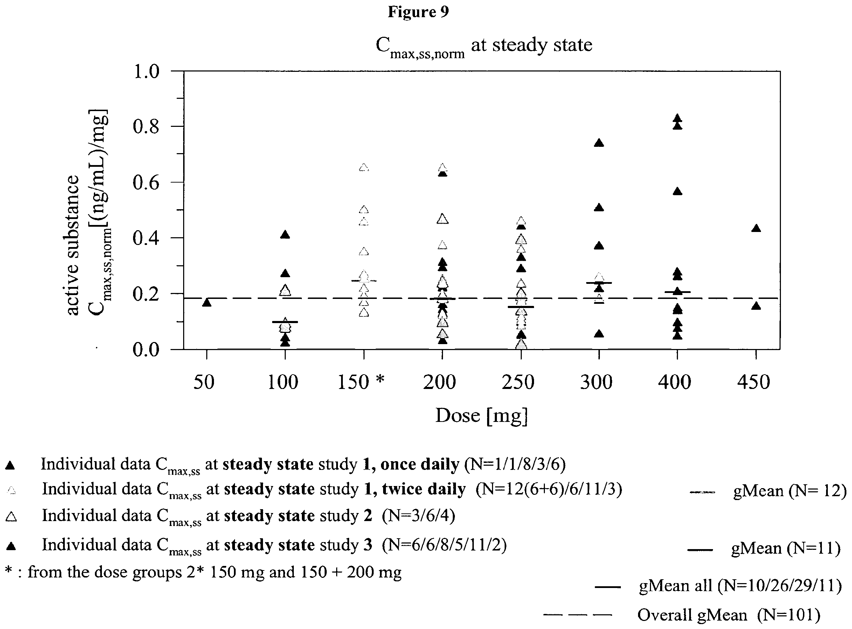

**Figure 9** **-** Individual and geometric mean dose-normalized maximum plasma concentrations at steady state of the compound 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate from three different Phase I trials in cancer patients, after administration of the active substance in a soft gelatin capsule dosage form.

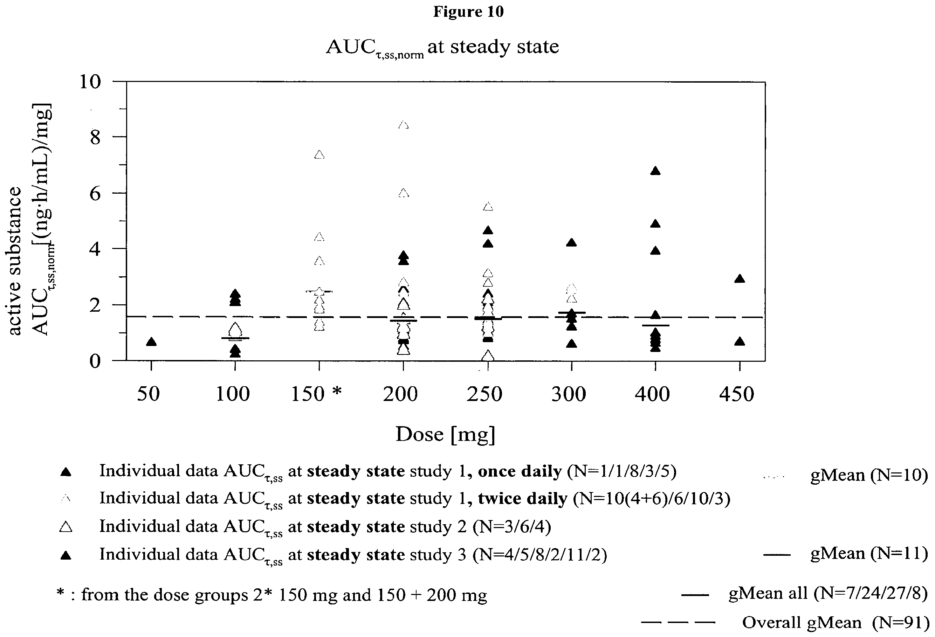

**Figure 10** **-** Individual and geometric mean dose-normalized area under the curve (AUC) values at steady state of the compound 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate from three different Phase I trials in cancer patients, after administration of the active substance in a soft gelatin capsule dosage form.

### Detailed description of the invention

The methods for measuring the dissolution rate in accordance with the present invention are according to European Pharmacopeia 6.2 and described in the following.

The dissolution tests use Apparatus 2 (paddle) according to European Pharmacopeia 6.2, with a spindle rotation speed of 100 rpm and a dissolution medium without additives of 0.1 M HCl, pH 1.0, at 37° C. The method is adjustable to a change in the medium volume. Further methods include a stirring speed of between 50 and 150 rpm, using Apparatus 1 or 2 according to European Pharmacopeia 6.2, a dissolution medium with a pH of between 1 and 6.8, a volume of between 500 and 2000 ml, optionally using sinkers, optionally in the presence of surfactants and/or enzymes, and optionally in the presence of organic solvents or using common simulated intestinal or gastric fluids. In other conditions, such as when changing the pH of the dissolution medium, as shown in Figure 6, the dissolution rate may be different. Hence, in accordance with the results of Figure 6, the dissolution rate may decrease with an increase of the pH. This may be due to a change in the pH dependent solubility of the active substance. In addition, in the presence of surfactants the dissolution rate may increase. Further variations of the dissolution test conditions, such as temperature, rotation speed, volume or Apparatus may influence the dissolution rate as well.

In accordance with the present invention, dissolution tests with Apparatus 2 (paddle), 100 rpm, 900 mL pH 1.0 (0.1 M HCl) dissolution medium and 37°C, indicate that the lecithin amounts in the formulation are able to increase the dissolution rate, in contrast to the formulation without lecithin (Fig. 2).

Further, in accordance with the present invention, it can be shown that the dissolution behaviour of the drug product is independent of the dosage strength. Fig. 5 shows this for soft gelatin capsule dosage forms.

In some instances, the measured dissolution rates with Apparatus 2 (paddle), 100 rpm, 900 mL pH 1.0 (0.1 M HCl) dissolution medium and 37°C, may show a significant difference in the dissolution behaviour of different batches of soft gelatin capsule pharmaceutical dosage forms. This is shown in Figure 7, for two different batches used in a Phase I human bioavailability study (Figure 8). As can be seen, for the measured values up to 60 minutes release time, batch A shows a faster release than batch B. However, this difference between the dissolution profile of different batches up to 60 minutes drug release observed with 100 rpm have no relevance on the *in vivo* pharmacokinetic behaviour of the active substance based on a immediate release formulation, as can be seen in Figure 8.

In the Phase I study (see Figure 9), the plasma concentrations of the active substance were measurable in a few subjects already 0.5 hours after drug administration and in most subjects one hour after drug administration. The plasma concentrations of the active substance increased up to about 2-4 hours after administration of the capsules to about 9 ng/mL (gMean value = geometric mean value) at a given dose of 150 mg to healthy subjects. Some subjects showed a second increase or a plateau in plasma concentrations of the active substance at about 4-6 hours. Afterwards the plasma concentration decreased in an at least bi-exponential manner. The plasma concentrations of the active substance were about 15% of the maximum plasma concentration 24 hours after administration and about 7-8% 48 hours after administration. About 2/3 of the subjects had measurable plasma concentrations of the active substance 48 h after drug administration. The variability of the plasma concentrations of the active substance at the different time points was high up to 2 hours (gCV: 100-250%) but moderate at later time points (gCV: 30-45%).

So far, the plasma concentrations of the active substance displayed high inter-patient variability in PK parameters in all trials, which prevented a formal statistical testing of dose-proportionality. However, in three Phase I trials in cancer patients with various advanced solid tumors there was no sign for a deviation from a dose proportional increase in AUC and Cₘₐₓ of the active substance observed through visual inspection neither after single dose nor at steady state for once and twice daily dosing (Figures 9 and 10). As a consequence, in cancer patients gMean C_{max,ss} and AUC_{τ,ss} of the active substance increased in a dose-proportional manner after single dose and at steady state, for qd and bid dosing. There was no deviation from dose-proportionality observed for drug plasma concentrations measured before drug administration at steady state (C_{pre,ss}) in cancer patients in various clinical trials, found through visual inspection.

In addition, in two Phase 2 trials of monotherapy with the active substance 3-Z-[1-(4-(N-((4-methyl-piperazin-1-yl)-methylcarbonyl)-N-methyl-amino)-anilino)-1-phenylmethylene]-6-methoxycarbonyl-2-indolinone-monoethanesulphonate administered as soft gelatin capsule dosage form in patients with non small-cell lung cancer (NSCLC) or hormone refractory prostate cancer (HRPC), there was no obvious deviation from a dose proportional increase in pre-dose plasma concentrations of the active substance of both dose groups tested (150 and 250 mg twice daily of the active substance) at steady state.

The dosage for oral administration for humans is preferably between 25 and 300 mg per administration, with one or more administrations per day.

However, it may sometimes be necessary to depart from the amounts specified, depending on the body weight, the route of administration, the individual response to the active substance, the nature of its formulation and the time or interval over which the active substance is administered. Thus, in some cases it may be sufficient to use less than the minimum dose given above, whereas in other cases the upper limit may have to be exceeded. When administering large amounts it may be advisable to divide them up into a number of smaller doses spread over the day.

### Assessment of quality

The performance of a formulation may be assessed by measuring its relative bioavailability, i.e. comparing its bioavailability with the bioavailability of an aqueous solution of the active substance. Thus, (lipid) suspensions may also show satisfactory exposure of the patient due to the adequate solubility of the active substance within physiological conditions.

In other respects, if an increase of the drug release of the drug product in the presence of surfactants is observed, it can be expected that the drug release of the drug product is improved as well under *in vivo* conditions when tensides out of the gastrointestinal tract are present.

A soft gelatin capsule including a liquid formulation comprising a viscous lipid suspension of 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate in medium chain triglycerides, hard fat and lecithin, according to the invention, meets the adequate bioavailability requirements for the desired dosage range tailored to treatment with the drug substance 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate. This formulation further meets the specific immediate release profile range providing an appropriate plasma concentration-time profile of the active principle which is the aim of the present invention.

An advantage of such soft gelatin capsule containing a lipid suspension is that the water uptake into the formulation is very unlikely. The dosage form is divided into three different compartments, namely (a) a hydrophilic capsule shell and (b) the hydrophobic carrier system in which (c) the slightly hygroscopic powder of active substance is suspended. Due to ambient moisture the content of water may vary within these different compartments. It will migrate by diffusion until an equilibrium state is reached. The water content may affect different properties of the drug product, such as the chemical stability of the active substance (predominantly via hydrolysis), the dissolution of the active substance, or the elasticity of the capsule shell. The water uptake in the present system is primarily in the capsule shell. This can be shown by water vapour sorption experiments as well as by the correlation of the mass gain with the softening of the capsule (shown in Figure 1). The water uptake does further not affect the chemical stability of the drug substance. This is confirmed by the stress stability studies of, for example, 1 month at 70°C, and by long-term (3 years) and accelerated (6 months) stability study results for the systems in accordance with the present invention.

Furthermore, studies have shown that there is no relevant mass increase or sticking problem for the capsules in accordance with the present invention when stored in tight packaging materials below 30°C. Thus, recommended packaging for such capsules are, for example, glass containers or flexible/hard plastic containers (e.g. HDPE bottles), aluminium blisters (e.g. alu/alu blisters), plastic blisters (e.g. PVC, PVDC or Aclar^{®}) optionally with an over-packaging of an aluminium pouch, or an aluminium pouch or double poly bag.

Generally, soft gelatin capsules have a capsule shell made of gelatin, one or more plasticizing agents, in particular glycerol, optionally further auxiliary materials, such as dyes, colorant pigments, flavouring agents, sugar, oligosaccharides or polysaccharides, and a capsule formulation (or capsule filling) containing a solvent, adjuvants and one or more pharmacologically active substances. The term gelatin as used herein includes not only unmodified gelatin as in the European Pharmacopeia but also modified gelatin, such as for example succinated gelatin.

In the above-mentioned lipid suspension formulation, the lipid carrier is medium chain triglycerides comprised within 10 and 70 weight % of the lipid suspension formulation. Suitable medium chain triglycerides may be the commercial product Miglyol 812^{®}, Miglyol 8100, Miglyol 818^{®}, Miglyol 829^{®} or Miglyol 840^{®}.

A thickener adjusts the viscosity of the suspension. It stabilizes the suspension system, ensures optimal processing and guarantees an adequate capsule quality, especially as far as content uniformity or dissolution behaviour are concerned.

In the above-mentioned suspension formulation, the thickener is hard fat comprised within the range of 1 to 30 weight% of the suspension formulation, most preferably within 10 and 30 weight%. The most suitable hard fats have a melting range of 30 °C to 44°C, most preferably a melting range of 33 °C to 40°C. Suitable commercially available products are Gelucire^{®} 33/01, Witepsol^{®} W35 or Softisan^{®} 378. The determination of the most suitable melting range for hard fats can be performed as shown in Figure 3, by measurement of the effect of the melting range of the hard fat on the *in-vitro* dissolution behaviour over time.

Lecithin is a common excipient for carrier-systems in soft gelatin capsules. It is used as a glidant of the highly concentrated suspension during encapsulation, prevents blocking of ducts and pumps and ensures high mass uniformity of the encapsulated formulation. Furthermore Lecithin acts as a surfactant, which may improve distribution of the formulation-droplets during *in-vitro* dissolution testing (compare Fig. 2) as well as *in-vivo* for drug resorption. Furthermore it may also improve wetting of the active substance crystals. Suitable lecithin may be the commercial product Topcithin^{®}.

Lecithin, up to a certain content, is useful to improve the dissolution behaviour of the finished capsules. Exceeding amounts do not show an additional benefit during *in-vitro* dissolution testing, as shown in Figure 2.

In the above-mentioned lipid suspension formulation, the amount of lecithin is comprised within the range of 0.1 to 10 weight% of the lipid suspension formulation, most preferably within 0.25 and 2.5 %.

Three carrier systems (the hydrophilic P3, lipophilic P1 and lipophilic with surfactants P2 semi-solid suspension formulations described in the foregoing) were tested for bioavailability in non-clinical studies and all of them were identified to be suitable options for an oral dosage form of the active substance 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate.

However, for reasons of bioavailability, as is evident from the results shown in Figure 4, lipid (lipophilic) suspension formulations comprising a viscous suspension of 3-Z-[1-(4-(N-((4-methyl-piperazin-1-yl)-methylcarbonyl)-N-methyl-amino)-anilino)-1-phenylmethylene]-6-methoxycarbonyl-2-indolinone-monoethanesulphonate in medium chain triglycerides, hard fat and lecithin are preferred.

Hence, Figure 4 shows the results of a comparison of the absolute bioavailability (BA in %) tested in the rat over 24 hours for the aqueous solution (S) versus different carrier systems (P1, P2 and P3) of the active substance 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate. The experiment is described in the following.

The table below shows the composition of the tested carrier systems (semi-solid suspension formulations). P2 and P3 are not according to the invention.

| **Formulation** | **P1** | **P2** | **P3** |
|---|---|---|---|
| **Ingredients** | [%]* | | |
| Active Substance | 43.48 | 42.19 | 31.75 |
| Triglycerides, Medium-Chain | 37.83 | 41.77 | -- |
| Hard fat | 18.26 | 12.66 | -- |
| Cremophor RH40 | -- | 2.95 | -- |
| Lecithin | 0.43 | 0.42 | -- |
| Glycerol 85% | -- | -- | 3.17 |
| Purified Water | -- | -- | 4.76 |
| Macrogol 600 | -- | -- | 58.10 |
| Macrogol 4000 | -- | -- | 2.22 |
| * slight deviations of the quantities towards 100 percent may be caused by rounding errors | | | |

The semi-solid suspensions are filled in hard gelatin capsules (Capsugel, no. Y0303490). Each capsule contains approximately 15 to 20 mg of the formulation. The capsules are applied to the rats with a special device similar to gavage. For comparison an aqueous solution containing 0.5 % Natrosol 250 HX is applied via gavage. For calculation of the absolute bioavailability an additional group of rats is dosed intravenously with the compound dissolved in 5% glucose solution (aqueous solution (S)). 5 male Han Wistar rats (strain: CrlGlxBrlHan:WI) are used per group. Blood sampling times were 0.5 h, 1 h, 2 h, 4 h, 8 h, 24 h post dose and plasma was analysed by a validated HPLC/MS/MS method. From the plasma level time curves areas under the curve (AUC) were calculated by linear trapezoidal rule. Dose normalised AUCs of the oral formulation are divided by dose normalised AUCs of the intravenous formulation for the calculation of the absolute bioavailability. As can be seen from the results of the experiment shown in Figure 4, the bioavailability is similar for the aqueous solution (S: 11%) and the different carrier systems of active substance (P1: 14%, P2: 10% and P3: 10%), however the inter-individual variation (standard deviation of bioavailability) is smaller for the aqueous solution (S) and the carrier system (P1) when compared to the carrier systems (P2) and (P3) (2.8 and 4.1 versus 7.4 and 7.1), indicating a practically complete relative bioavailability for the tested formulations (P1, P2 and P3) versus the solution (S) but a higher variation in the carrier systems (P2) and (P3).

The lipid suspension formulation as hereinbefore described may be part of a capsule pharmaceutical dosage form consisting of a capsule shell and a capsule formulation (or capsule filling), in which the capsule formulation (or capsule filling) comprises the lipid suspension formulation as hereinbefore described according to the invention. The capsule pharmaceutical dosage form may be a soft gelatin capsule, a hard gelatin capsule, or an hydroxypropylmethylcellulose (HPMC) capsule, a polyvinyl alcohol polymer capsule or a pullulan capsule.

In the case of a hard gelatin capsule or an hydroxypropylmethylcellulose (HPMC) capsule, a polyvinyl alcohol polymer capsule or a pullulan capsule, the filled capsule may further be sealed or banded.

Preferably, the capsule is a soft gelatin capsule consisting of a capsule shell comprising gelatin, one or more plasticizing agents and optionally further auxiliary materials, and a capsule formulation (or capsule filling), characterized in that the capsule formulation (or capsule filling) comprises the lipid suspension formulation as hereinbefore described according to the invention.

The capsule pharmaceutical dosage form, and especially the soft gelatin capsules, may be stored in suitable glass containers or in flexible/hard plastic containers, preferably non-PVC materials based, or in plastic (e.g. PVC, PVDC or Aclar^{®}) blisters optionally with an over-packaging of aluminium (aluminium pouch), or in aluminium blisters consisting of e.g a bottom foil of PA/Al/PVC and an aluminium lidding foil, the later providing the highest water protection. Hence, the containers may be designed so as to provide particular protection for the capsule pharmaceutical dosage form, and especially the soft gelatin capsules, e.g. to protect them from light, oxygen or water. Flexible plastic containers may contain additional protection, e.g. in the form of an additional aluminium packaging.

The capsule pharmaceutical dosage form may be prepared by conventional methods of producing capsules known from the literature. The soft gelatin capsule may be prepared by conventional methods of producing soft gelatin capsules known from the literature, such as for example the "rotary die procedure", described for example in Swarbrick, Boylann, Encyclopedia of pharmaceutical technology, Marcel Dekker, 1990, Vol. 2, pp 269ff or in Lachmann et al., "The Theory and Practice of Industrial Pharmacy", 2nd Edition, pages 404-419, 1976, or other procedures, such as those described for example in Jimerson R. F. et al., "Soft gelatin capsule update", Drug Dev. Ind. Pharm., Vol. 12, No. 8-9, pp. 1133-44, 1986.

The lipid suspension formulation may be prepared by conventional methods of producing formulations known from the literature, i.e. by mixing the ingredients at a pre-determined temperature in a pre-determined order in order to obtain a homogenized suspension.

Alternatively, the lipid suspension formulation may be prepared in accordance with the procedure described in Example 10.

Lipid suspension formulation of the active substance, finished soft gelatin capsules containing same and packaging materials for the packaging of finished soft gelatin capsules are illustrated by the Examples and Figures that follow. The Examples serve purely as an illustration and are not to be construed in a limiting capacity.

### Examples of carrier systems (formulations), soft gelatin capsules, packaging materials, and of a manufacturing process for the preparation of a lipid suspension formulation of the active substance

The active substance in all the Examples is 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate.

### Example 1

### Lipid based carrier system

| **Formulation** | **A** | **B** | **C** |
|---|---|---|---|
| **Ingredients** | [%]* | | |
| Active Substance | 43.48 | 43.48 | 43.48 |
| Triglycerides, Medium-Chain | 28.70 | 37.83 | 38.045 |
| Hard fat | 27.39 | 18.26 | 18.26 |
| Lecithin | 0.43 | 0.43 | 0.215 |
| * slight deviations of the quantities towards 100 percent may be caused by rounding errors | | | |

### Reference Example 2

### Lipid based carrier system with additional surfactant

| **Ingredients** | **[%]*** |
|---|---|
| Active Substance | 42.19 |
| Triglycerides, Medium-Chain | 41.77 |
| Hard fat | 12.66 |
| Cremophor RH40 | 2.95 |
| Lecithin | 0.42 |
| * slight deviations of the quantities towards 100 percent may be caused by rounding errors | |

### Reference Example 3

### Hydrophilic carrier system

| **Ingredients** | **[%]*** |
|---|---|
| Active Substance | 31.75 |
| Glycerol 85% | 3.17 |
| Purified Water | 4.76 |
| Macrogol 600 | 58.10 |
| Macrogol 4000 | 2.22 |
| * slight deviations of the quantities towards 100 percent may be caused by rounding errors | |

### Example 4

### Soft gelatin capsule containing 50 mg of active substance

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 60.20 | 60.20 | 60.20 |
| Triglycerides, Medium-chain | Carrier | 40.95 | 53.70 | 54.00 |
| Hard fat | Thickener | 38.25 | 25.50 | 25.50 |
| Lecithin | Wetting agent / Glidant | 0.60 | 0.60 | 0.30 |
| Gelatin | Film-former | 72.25 | 72.25 | 72.25 |
| Glycerol 85% | Plasticizer | 32.24 | 32.24 | 32.24 |
| Titanium dioxide | Colorant | 0.20 | 0.20 | 0.20 |
| Iron oxide A | Colorant | 0.32 | 0.32 | 0.32 |
| Iron oxide B | Colorant | 0.32 | 0.32 | 0.32 |
| **Total Capsule Weight** | | **245.33** | **245.33** | 245.33 |
| * The figures refer to the amount of ethanesulfonate salt (dry basis) equivalent to the labeled amount of the free base | | | | |

### Example 4a

### Soft gelatin capsule containing 75 mg of active substance

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 90.3 | 90.3 | 90.3 |
| Triglycerides, Medium-chain | Carrier | 61.425 | 80.55 | 80.1 |
| Hard fat | Thickener | 57.375 | 38.25 | 38.25 |
| Lecithin | Wetting agent / Glidant | 0.9 | 0.9 | 1.35 |
| Gelatin | Film-former | 107.11 | 107.11 | 107.11 |
| Glycerol 85% | Plasticizer | 46.84 | 46.84 | 46.84 |
| Titanium dioxide | Colorant | 0.35 | 0.35 | 0.35 |
| Iron oxide A | Colorant | 0.058 | 0.058 | 0.058 |
| Iron oxide B | Colorant | 0.16 | 0.16 | 0.16 |
| **Total Capsule Weight** | | **364.518** | **364.518** | **364.518** |
| * The figures refer to the amount of ethanesulfonate salt (dry basis) equivalent to the labeled amount of the free base | | | | |

### Example 5

### Soft gelatin capsule containing 100 mg of active substance

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 120.40 | 120.40 | 120.40 |
| Triglycerides, Medium-chain | Carrier | 81.90 | 107.40 | 106.8 |
| Hard fat | Thickener | 76.50 | 51.00 | 51.00 |
| Lecithin | Wetting agent / Glidant | 1.20 | 1.20 | 1.80 |
| Gelatin | Film-former | 111.58 | 111.58 | 111.58 |
| Glycerol 85% | Plasticizer | 48.79 | 48.79 | 48.79 |
| Titanium dioxide | Colorant | 0.36 | 0.36 | 0.36 |
| Iron oxide A | Colorant | 0.06 | 0.06 | 0.06 |
| Iron oxide B | Colorant | 0.17 | 0.17 | 0.17 |
| **Total Capsule Weight** | | **440.96** | **440.96** | **440.96** |
| * The figures refer to the amount of ethanesulfonate salt (dry basis) equivalent to the labeled amount of the free base | | | | |

### Example 6

### Soft gelatin capsule containing 125 mg of active substance

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 150.50 | 150.50 | 150.50 |
| Triglycerides, Medium-chain | Carrier | 102.375 | 134.25 | 133.5 |
| Hard fat | Thickener | 95.625 | 63.75 | 63.75 |
| Lecithin | Wetting agent / Glidant | 1.50 | 1.50 | 2.25 |
| Gelatin | Film-former | 142.82 | 142.82 | 142.82 |
| Glycerol 85% | Plasticizer | 62.45 | 62.45 | 62.45 |
| Titanium dioxide | Colorant | 0.47 | 0.47 | 0.47 |
| Iron oxide A | Colorant | 0.08 | 0.08 | 0.08 |
| Iron oxide B | Colorant | 0.22 | 0.22 | 0.22 |
| **Total Capsule Weight** | | **556.04** | **556.04** | **556.04** |
| * The figures refer to the amount of ethanesulfonate salt (dry basis) equivalent to the labeled amount of the free base | | | | |

### Example 7

### Soft gelatin capsule containing 150 mg of active substance

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 180.60 | 180.60 | 180.60 |
| Triglycerides, Medium-chain | Carrier | 122.85 | 161.10 | 160.20 |
| Hard fat | Thickener | 114.75 | 76.50 | 76.50 |
| Lecithin | Wetting agent / Glidant | 1.80 | 1.80 | 2.70 |
| Gelatin | Film-former | 142.82 | 142.82 | 142.82 |
| Glycerol 85% | Plasticizer | 62.45 | 62.45 | 62.45 |
| Titanium dioxide | Colorant | 0.47 | 0.47 | 0.47 |
| Iron oxide A | Colorant | 0.08 | 0.08 | 0.08 |
| Iron oxide B | Colorant | 0.22 | 0.22 | 0.22 |
| **Total Capsule Weight** | | **626.04** | **626.04** | **626.04** |
| * The figures refer to the amount of ethanesulfonate salt (dry basis) equivalent to the labeled amount of the free base | | | | |

### Example 8

### Soft gelatin capsule containing 200 mg of active substance

| | | **Formulation A** | **Formulation B** | **Formulation C** |
|---|---|---|---|---|
| **Ingredients** | **Function** | **mg per capsule** | **mg per capsule** | **mg per capsule** |
| Active Substance* | Active Ingredient | 240.80 | 240.80 | 240.80 |
| Triglycerides, Medium-chain | Carrier | 163.30 | 214.80 | 216.00 |
| Hard fat | Thickener | 153.50 | 102.00 | 102.00 |
| Lecithin | Wetting agent / Glidant | 2.40 | 2.40 | 1.20 |
| Gelatin | Film-former | 203.19 | 203.19 | 203.19 |
| Glycerol 85% | Plasticizer | 102.61 | 102.61 | 102.61 |
| Titanium dioxide | Colorant | 0.57 | 0.57 | 0.57 |
| Iron oxide A | Colorant | 0.90 | 0.90 | 0.90 |
| Iron oxide B | Colorant | 0.90 | 0.90 | 0.90 |
| **Total Capsule Weight** | | **868.17** | **868.17** | **868.17** |
| * The figures refer to the amount of ethanesulfonate salt (dry basis) equivalent to the labeled amount of the free base | | | | |

### Example 9

Packaging materials for the packaging of the soft gelatin capsules of above examples 4 to 8 may be glass containers, flexible/hard plastic containers or PVC/PVDC blisters, optionally within an aluminium pouch, or alu/alu blisters.

### Example 10

In the following, a manufacturing process for the preparation of a lipid suspension formulation of the active substance and a process for the encapsulation are described.
a: Hard fat and parts of Medium-chain triglycerides are pre-mixed in the processing unit. Subsequently lecithin, the rest of medium-chain triglycerides and the active substance are added. The suspension is mixed, homogenized, deaerated and finally sieved to produce the formulation (Fillmix).
b. The gelatin basic mass components (glycerol, water and gelatin) are mixed and dissolved at elevated temperature. Then, the corresponding colours are added and mixed, producing the Coloured Gelatin Mass.
c. After adjustment of the encapsulation machine, Fillmix and Coloured Gelatin Mass are processed into soft gelatin capsules using the rotary-die process. This process is e.g. described in Swarbrick, Boylann, Encyclopedia of pharmaceutical technology, Marcel Dekker, 1990, Vol. 2, pp 269 ff.
d. The initial drying is carried out using a rotary dryer. For the final drying step, capsules are placed on trays. Drying is performed at 15 - 26°C and low relative humidity.
e. After 100% visual inspection of the capsules for separation of deformed or leaking capsules, the capsules are size sorted.
f. Finally, the capsules are imprinted, using an Offset printing technology or an Ink-jet printing technology. Alternatively, the capsule imprint can be made using the Ribbon printing technology, a technology in which the gelatin bands are imprinted prior to the encapsulation step c.

## Claims

1. Pharmaceutical dosage form of the active substance 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethylsulphonate which delivers an immediate release profile in which not less than 70% (Q65%) of the active substance is dissolved in 60 minutes in vitro under the following in vitro dissolution conditions according to European Pharmacopeia 6.2: Apparatus 2 (paddle), dissolution medium with 0.1 M HCl (pH 1) and stirring speed of 50 to 150 rpm, at a temperature of 37° C and which comprises a viscous lipid suspension formulation of 10 to 50 weight% of the active substance in 10 to 70 weight% of medium chain triglycerides, 1 to 30 weight% of hard fat and 0.1 to 10 weight% of lecithin, based on the total weight of the viscous lipid suspension formulation.

2. Pharmaceutical dosage form according to claim 1, comprising dose-range values of between 25 to 300 mg of the active substance.

3. Pharmaceutical dosage form according to claim 1, comprising 10 to 70 weight % of medium chain triglycerides, 10 to 30 weight % of hard fat and 0.25 to 2.5 weight % of lecithin.

4. Pharmaceutical dosage form according to any one of claims 1 to 3, wherein the dosage form is suitable for oral delivery.

5. Pharmaceutical dosage form according to claim 4 in form of a soft gelatin capsule containing 50 mg of active substance free base equivalent, selected from the following compositions A, B and C:

6. | | **Formulation A** | **Formulation B** | **Formulation C** |
|---|---|---|---|
| **Ingredients** | **mg per capsule** | **mg per capsule** | **mg per capsule** |
| Active Substance | 60.20 | 60.20 | 60.20 |
| Triglycerides, Medium-chain | 40.95 | 53.70 | 54.00 |
| Hard fat | 38.25 | 25.50 | 25.50 |
| Lecithin | 0.60 | 0.60 | 0.30 |
| Gelatin | 72.25 | 72.25 | 72.25 |
| Glycerol 85% | 32.24 | 32.24 | 32.24 |
| Titanium dioxide | 0.20 | 0.20 | 0.20 |
| Iron oxide A | 0.32 | 0.32 | 0.32 |
| Iron oxide B | 0.32 | 0.32 | 0.32 |
| **Total Capsule Weight** | **245.33** | **245.33** | 245.33 |
Pharmaceutical dosage form according to claim 4 in form of a soft gelatin capsule containing 75 mg of active substance free base equivalent, selected from the following compositions A, B and C:
| | **Formulation A** | **Formulation B** | **Formulation C** |
|---|---|---|---|
| **Ingredients** | **mg per capsule** | **mg per capsule** | **mg per capsule** |
| Active Substance | 90.3 | 90.3 | 90.3 |
| Triglycerides, Medium-chain | 61.425 | 80.55 | 80.1 |
| Hard fat | 57.375 | 38.25 | 38.25 |
| Lecithin | 0.9 | 0.9 | 1.35 |
| Gelatin | 107.11 | 107.11 | 107.11 |
| Glycerol 85% | 46.84 | 46.84 | 46.84 |
| Titanium dioxide | 0.35 | 0.35 | 0.35 |
| Iron oxide A | 0.058 | 0.058 | 0.058 |
| Iron oxide B | 0.16 | 0.16 | 0.16 |
| **Total Capsule Weight** | **364.518** | **364.518** | **364.518** |

7. Pharmaceutical dosage form according to claim 4 in form of a soft gelatin capsule containing 100 mg of active substance free base equivalent, selected from the following compositions A, B and C:
| | **Formulation A** | **Formulation B** | **Formulation C** |
|---|---|---|---|
| **Ingredients** | **mg per capsule** | **mg per capsule** | **mg per capsule** |
| Active Substance | 120.40 | 120.40 | 120.40 |
| Triglycerides, Medium-chain | 81.90 | 107.40 | 106.8 |
| Hard fat | 76.50 | 51.00 | 51.00 |
| Lecithin | 1.20 | 1.20 | 1.80 |
| Gelatin | 111.58 | 111.58 | 111.58 |
| Glycerol 85% | 48.79 | 48.79 | 48.79 |
| Titanium dioxide | 0.36 | 0.36 | 0.36 |
| Iron oxide A | 0.06 | 0.06 | 0.06 |
| Iron oxide B | 0.17 | 0.17 | 0.17 |
| **Total Capsule Weight** | **440.96** | **440.96** | **440.96** |

8. Pharmaceutical dosage form according to claim 4 in form of a soft gelatin capsule containing 125 mg of active substance free base equivalent, selected from the following compositions A, B and C:
| | **Formulation A** | **Formulation B** | **Formulation C** |
|---|---|---|---|
| **Ingredients** | **mg per capsule** | **mg per capsule** | **mg per capsule** |
| Active Substance | 150.50 | 150.50 | 150.50 |
| Triglycerides, Medium-chain | 102.375 | 134.25 | 133.5 |
| Hard fat | 95.625 | 63.75 | 63.75 |
| Lecithin | 1.50 | 1.50 | 2.25 |
| Gelatin | 142.82 | 142.82 | 142.82 |
| Glycerol 85% | 62.45 | 62.45 | 62.45 |
| Titanium dioxide | 0.47 | 0.47 | 0.47 |
| Iron oxide A | 0.08 | 0.08 | 0.08 |
| Iron oxide B | 0.22 | 0.22 | 0.22 |
| **Total Capsule Weight** | **556.04** | **556.04** | **556.04** |

9. Pharmaceutical dosage form according to claim 4 in form of a soft gelatin capsule containing 150 mg of active substance free base equivalent, selected from the following compositions A, B and C:
| | **Formulation A** | **Formulation B** | **Formulation C** |
|---|---|---|---|
| **Ingredients** | **mg per capsule** | **mg per capsule** | **mg per capsule** |
| Active Substance | 180.60 | 180.60 | 180.60 |
| Triglycerides, Medium-chain | 122.85 | 161.10 | 160.20 |
| Hard fat | 114.75 | 76.50 | 76.50 |
| Lecithin | 1.80 | 1.80 | 2.70 |
| Gelatin | 142.82 | 142.82 | 142.82 |
| Glycerol 85% | 62.45 | 62.45 | 62.45 |
| Titanium dioxide | 0.47 | 0.47 | 0.47 |
| Iron oxide A | 0.08 | 0.08 | 0.08 |
| Iron oxide B | 0.22 | 0.22 | 0.22 |
| **Total Capsule Weight** | **626.04** | **626.04** | **626.04** |

10. Pharmaceutical dosage form according to claim 4 in form of a soft gelatin capsule containing 200 mg of active substance free base equivalent, selected from the following compositions A, B and C:
| | **Formulation A** | **Formulation B** | **Formulation C** |
|---|---|---|---|
| **Ingredients** | **mg per capsule** | **mg per capsule** | **mg per capsule** |
| Active Substance | 240.80 | 240.80 | 240.80 |
| Triglycerides, Medium-chain | 163.30 | 214.80 | 216.00 |
| Hard fat | 153.50 | 102.00 | 102.00 |
| Lecithin | 2.40 | 2.40 | 1.20 |
| Gelatin | 203.19 | 203.19 | 203.19 |
| Glycerol 85% | 102.61 | 102.61 | 102.61 |
| Titanium dioxide | 0.57 | 0.57 | 0.57 |
| Iron oxide A | 0.90 | 0.90 | 0.90 |
| Iron oxide B | 0.90 | 0.90 | 0.90 |
| **Total Capsule Weight** | **868.17** | **868.17** | **868.17** |

## Patentansprüche

1. Pharmazeutische Dosierungsform der aktiven Substanz bzw. Wirksubstanz 3-Z-[1-(4-(N-((4-Methyl-piperazin-1-yl)-methylcarbonyl)-N-methyl-amino)-anilino)-1-phenyl-methylen]-6-methoxycarbonyl-2-indolinon-monoethansulphonat, die ein sofortiges Freisetzungsprofil bereitstellt, in dem nicht weniger als 70% (Q65%) der aktiven Substanz bzw. Wirksubstanz in 60 Minuten in vitro unter den nachfolgenden in vitro-Lösungsbedingungen gemäß der European Pharmacopeia 6.2 gelöst werden: Vorrichtung 2 (Paddle bzw. Blattrührer), Lösungsmedium mit 0,1 M HCl (pH 1) und einer Rührgeschwindigkeit von 50 bis 150 U/min, bei einer Temperatur von 37°C, und das umfasst eine viskose Lipid-Suspensionsformulierung von 10 bis 50 Gew-% Wirksubstanz bzw. aktive Substanz in 10 bis 70 Gew-% Triglyceride mittlerer Kettenlänge, 1 bis 30 Gew.-% Hartfett und 0,1 bis 10 Gew.-% Lecithin, basierend auf dem Gesamtgewicht der viskosen Lipid-Suspensionsformulierung.

2. Pharmazeutische Dosierungsform nach Anspruch 1, umfassend Dosisbereichswerte zwischen 25 und 300 mg der Wirksubstanz bzw. aktiven Substanz.

3. Pharmazeutische Dosierungsform nach Anspruch 1, umfassend 10 bis 70 Gew.-% Triglyceride mittlerer Kettenlänge, 10 bis 30 Gew.-% Hartfett und 0,25 bis 2,5 Gew.-% Lecithin.

4. Pharmazeutische Dosierungsform nach irgendeinem der Ansprüche 1 bis 3, wobei die Dosierungsform für die orale Anwendung bzw. Verabreichung geeignet ist.

5. Pharmazeutische Dosierungsform nach Anspruch 4 in Form einer Weichgelatinekapsel, enthaltend 50 mg Wirksubstanz bzw. aktive Substanz, äquivalent zur freien Base, ausgewählt aus den nachfolgenden Zusammensetzungen A, B und C:
| | **Formulierung A** | **Formulierung B** | **Formulierung C** |
|---|---|---|---|
| **Bestandteile** | **mg pro Kapsel** | **mg pro Kapsel** | **mg pro Kapsel** |
| Wirksubstanz bzw. aktive Substanz | 60,20 | 60,20 | 60,20 |
| Triglyceride, mittlere Kettenlänge | 40.95 | 53,70 | 54,00 |
| Hartfett | 38,25 | 25,50 | 25,50 |
| Lecithin | 0,60 | 0,60 | 0,30 |
| Gelatine | 72,25 | 72,25 | 72,25 |
| Glycerol 85% | 32,24 | 32,24 | 32,24 |
| Titandioxid | 0,20 | 0,20 | 0,20 |
| Eisenoxid A | 0,32 | 0,32 | 0,32 |
| Eisenoxid B | 0,32 | 0,32 | 0,32 |
| **Gesamtkapselgewicht** | **245,33** | **245,33** | 245,33 |

6. Pharmazeutische Dosierungsform nach Anspruch 4 in Form einer Weichgelatinekapsel, enthaltend 75 mg Wirksubstanz, äquivalent zur freien Base, ausgewählt aus den nachfolgenden Zusammensetzungen A, B und C:
| | **Formulierung A** | **Formulierung B** | **Formulierung C** |
|---|---|---|---|
| **Bestandteile** | **mg pro Kapsel** | **mg pro Kapsel** | **mg pro Kapsel** |
| Wirksubstanz bzw. aktive Substanz | 90,3 | 90,3 | 90,3 |
| Triglyceride, mittlere Ketten länge | 61,425 | 80,55 | 80,1 |
| Hartfett | 57,375 | 38,25 | 38,25 |
| Lecithin | 0.9 | 0,9 | 1,35 |
| Gelatine | 107,1 1 | 107,11 | 107,11 |
| Glycerol 85% | 46,84 | 46,84 | 46,84 |
| Titandioxid | 0,35 | 0,35 | 0,35 |
| Eisenoxid A | 0,058 | 0,058 | 0,058 |
| Eisenoxid B | 0,16 | 0,16 | 0,16 |
| **Gesamtkapselgewicht** | **364,518** | **364,518** | **364,518** |

7. Pharmazeutische Dosierungsform nach Anspruch 4 in Form einer Weichgelatinekapsel, enthaltend 100 mg Wirksubstanz, äquivalent zur freien Base, ausgewählt aus den nachfolgenden Zusammensetzungen A, B und C:
| | **Formulierung A** | **Formulierung B** | **Formulierung C** |
|---|---|---|---|
| **Bestandteile** | **mg pro Kapsel** | **mg pro Kapsel** | **mg pro Kapsel** |
| Wirksubstanz bzw. aktive Substanz | 120,40 | 120,40 | 120,40 |
| Triglyceride, mittlere Kettenlänge | 81,90 | 107,40 | 106,8 |
| Hartfett | 76,50 | 51,00 | 51,00 |
| Lecithin | 1,20 | 1,20 | 1,80 |
| Gelatine | 111,58 | 111,58 | 111,58 |
| Glycerol 85% | 48,79 | 48,79 | 48,79 |
| Titandioxid | 0,36 | 0,36 | 0,36 |
| Eisenoxid A | 0,06 | 0,06 | 0,06 |
| Eisenoxid B | 0,17 | 0,17 | 0,17 |
| **Gesamtkapselgewicht** | **440,96** | **440,96** | **440,96** |

8. Pharmazeutische Dosierungsform nach Anspruch 4 in Form einer Weichgelatinekapsel, enthaltend 125 mg Wirksubstanz, äquivalent zur freien Base, ausgewählt aus den nachfolgenden Zusammensetzungen A, B und C:
| | **Formulierung A** | **Formulierung B** | **Formulierung C** |
|---|---|---|---|
| **Bestandteile** | **mg pro Kapsel** | **mg pro Kapsel** | **mg pro Kapsel** |
| Wirksubstanz bzw. aktive Substanz | 150,50 | 150,50 | 150,50 |
| Triglyceride, mittlere Kettenlänge | 102,375 | 134,25 | 133,5 |
| Hartfett | 95,625 | 63,75 | 63,75 |
| Lecithin | 1,50 | 1,50 | 2,25 |
| Gelatine | 142,82 | 142,82 | 142,82 |
| Glycerol 85% | 62,45 | 62,45 | 62,45 |
| Titandioxid | 0,47 | 0,47 | 0,47 |
| Eisenoxid A | 0,08 | 0,08 | 0,08 |
| Eisenoxid B | 0,22 | 0,22 | 0,22 |
| **Gesamtkapselgewicht** | **556,04** | **556,04** | **556,04** |

9. Pharmazeutische Dosierungsform nach Anspruch 4 in Form einer Weichgelatinekapsel, enthaltend 150 mg Wirksubstanz, äquivalent zur freien Base, ausgewählt aus den nachfolgenden Zusammensetzungen A, B und C:
| | **Formulierung A** | **Formulierung B** | **Formulierung C** |
|---|---|---|---|
| **Bestandteile** | **mg pro Kapsel** | **mg pro Kapsel** | **mg pro Kapsel** |
| Wirksubstanz bzw. aktive Substanz | 180,60 | 180,60 | 180,60 |
| Triglyceride, mittlere Kettenlänge | 122,85 | 161,10 | 160,20 |
| Hartfett | 114,75 | 76,50 | 76,50 |
| Lecithin | 1,80 | 1,80 | 2,70 |
| Gelatine | 142,82 | 142,82 | 142,82 |
| Glycerol 85% | 62,45 | 62,45 | 62,45 |
| Titandioxid | 0,47 | 0,47 | 0,47 |
| Eisenoxid A | 0,08 | 0,08 | 0.08 |
| Eisenoxid B | 0,22 | 0,22 | 0,22 |
| **Gesamtkapselgewicht** | **626,04** | **626,04** | **626,04** |

10. Pharmazeutische Dosierungsform nach Anspruch 4 in Form einer Weichgelatinekapsel, enthaltend 200 mg Wirksubstanz, äquivalent zur freien Base, ausgewählt aus den nachfolgenden Zusammensetzungen A, B und C:
| | **Formulierung A** | **Formulierung B** | **Formulierung C** |
|---|---|---|---|
| **Bestandteile** | **mg pro Kapsel** | **mg pro Kapsel** | **mg pro Kapsel** |
| Wirksubstanz bzw. aktive Substanz | 240,80 | 240,80 | 240,80 |
| Triglyceride, mittlere Kettenlängc | 163,30 | 214,80 | 216,00 |
| Hartfett | 153,50 | 102,00 | 102,00 |
| Lecithin | 2,40 | 2,40 | 1,20 |
| Gelatine | 203,19 | 203,19 | 203,19 |
| Glycerol 85% | 102,61 | 102,61 | 102,61 |
| Titandioxid | 0,57 | 0,57 | 0,57 |
| Eisenoxid A | 0,90 | 0,90 | 0,90 |
| Eisenoxid B | 0,90 | 0,90 | 0,90 |
| **Gesamtkapselgewicht** | **868,17** | **868,17** | **868,17** |

## Revendications

1. Forme posologique pharmaceutique du principe actif 3-Z-[1-(4-(N-((4-méthyl-pipérazin-1-yl)-méthylcarbonyl)-N-méthyl-amino)-anilino)-1-phényl-méthylène]-6-méthoxycarbonyl-2-indolinone-monoéthylsulfonate qui fournit un profil de libération immédiate dans lequel pas moins de 70 % (Q65 %) du principe actif est dissous en 60 minutes in vitro dans les conditions de dissolution in vitro suivantes selon la pharmacopée européenne 6.2 : Appareil 2 (palette), milieu de dissolution avec 0,1 M de HCl (pH 1) et vitesse d'agitation de 50 à 150 tr/m, à une température de 37 °C et qui comprend une formulation de suspension de lipide visqueux de 10 à 50 % en poids du principe actif dans 10 à 70 % en poids de triglycérides à chaîne moyenne, 1 à 30 % en poids de graisse dure et 0,1 à 10 % en poids de lécithine, sur la base du poids total de la formulation de suspension de lipide visqueux.

2. Forme posologique pharmaceutique selon la revendication 1, comprenant des valeurs de fourchette thérapeutique comprises entre 25 et 300 mg du principe actif.

3. Forme posologique pharmaceutique selon la revendication 1, comprenant de 10 à 70 % en poids de triglycérides à chaîne moyenne, de 10 à 30 % en poids de graisse dure et de 0,25 à 2,5 % en poids de lécithine.

4. Forme posologique pharmaceutique selon l'une quelconque des revendications 1 à 3, dans laquelle la forme posologique est adaptée à une administration orale.

5. Forme posologique pharmaceutique selon la revendication 4 sous la forme d'une capsule de gélatine molle contenant 50 mg d'équivalent de base libre de principe actif, sélectionné parmi les compositions A, B et C suivantes :
| | **Formulation A** | **Formulation B** | **Formulation C** |
|---|---|---|---|
| **Ingrédients** | **mg par capsule** | **mg par capsule** | **mg par capsule** |
| Principe actif | 60,20 | 60,20 | 60,20 |
| Triglycérides, chaîne moyenne | 40,95 | 53,70 | 54,00 |
| Graisse dure | 38,25 | 25,50 | 25,50 |
| Lécithine | 0,60 | 0,60 | 0,30 |
| Gélatine | 72,25 | 72,25 | 72,25 |
| Glycérol 85 % | 32,24 | 32,24 | 32,24 |
| Dioxyde de titane | 0,20 | 0,20 | 0,20 |
| Oxyde de fer A | 0,32 | 0,32 | 0,32 |
| Oxyde de fer B | 0,32 | 0,32 | 0,32 |
| **Poids total de la capsule** | **245,33** | **245,33** | 245,33 |

6. Forme posologique pharmaceutique selon la revendication 4 sous la forme d'une capsule de gélatine molle contenant 75 mg d'équivalent de base libre de principe actif, sélectionné parmi les compositions A, B et C suivantes :
| | **Formulation A** | **Formulation B** | **Formulation C** |
|---|---|---|---|
| **Ingrédients** | **mg par capsule** | **mg par capsule** | **mg par capsule** |
| Principe actif | 90,3 | 90,3 | 90,3 |
| Triglycérides, chaîne moyenne | 61,425 | 80,55 | 80,1 |
| Graisse dure | 57,375 | 38,25 | 38,25 |
| Lécithine | 0,9 | 0,9 | 1,35 |
| Gélatine | 107,11 | 107,11 | 107,11 |
| Glycérol 85 % | 46,84 | 46,84 | 46,84 |
| Dioxyde de titane | 0,35 | 0,35 | 0,35 |
| Oxyde de fer A | 0,058 | 0,058 | 0,058 |
| Oxyde de fer B | 0,16 | 0,16 | 0,16 |
| **Poids total de la capsule** | **364,518** | **364,518** | **364,518** |

7. Forme posologique pharmaceutique selon la revendication 4 sous la forme d'une capsule de gélatine molle contenant 100 mg d'équivalent de base libre de principe actif, sélectionné parmi les compositions A, B et C suivantes :
| | **Formulation A** | **Formulation B** | **Formulation C** |
|---|---|---|---|
| **Ingrédients** | **mg par capsule** | **mg par capsule** | **mg par capsule** |
| Principe actif | 120,40 | 120,40 | 120,40 |
| Triglycérides, chaîne moyenne | 81,90 | 107,40 | 106,8 |
| Graisse dure | 76,50 | 51,00 | 51,00 |
| Lécithine | 1,20 | 1,20 | 1,80 |
| Gélatine | 111,58 | 111,58 | 111,58 |
| Glycérol 85 % | 48,79 | 48,79 | 48,79 |
| Dioxyde de titane | 0,36 | 0,36 | 0,36 |
| Oxyde de fer A | 0,06 | 0,06 | 0,06 |
| Oxyde de fer B | 0,17 | 0,17 | 0,17 |
| **Poids total de la capsule** | **440,96** | **440,96** | **440,96** |

8. Forme posologique pharmaceutique selon la revendication 4 sous la forme d'une capsule de gélatine molle contenant 125 mg d'équivalent de base libre de principe actif, sélectionné parmi les compositions A, B et C suivantes :
| | **Formulation A** | **Formulation B** | **Formulation C** |
|---|---|---|---|
| **Ingrédients** | **mg par capsule** | **mg par capsule** | **mg par capsule** |
| Principe actif | 150,50 | 150,50 | 150,50 |
| Triglycérides, chaîne moyenne | 102,375 | 134,25 | 133,5 |
| Graisse dure | 95,625 | 63,75 | 63,75 |
| Lécithine | 1,50 | 1,50 | 2,25 |
| Gélatine | 142,82 | 142,82 | 142,82 |
| Glycérol 85 % | 62,45 | 62,45 | 62,45 |
| Dioxyde de titane | 0,47 | 0,47 | 0,47 |
| Oxyde de fer A | 0,08 | 0,08 | 0,08 |
| Oxyde de fer B | 0,22 | 0,22 | 0,22 |
| **Poids total de la capsule** | **556,04** | **556,04** | **556,04** |

9. Forme posologique pharmaceutique selon la revendication 4 sous la forme d'une capsule de gélatine molle contenant 150 mg d'équivalent de base libre de principe actif, sélectionné parmi les compositions A, B et C suivantes :
| | **Formulation A** | **Formulation B** | **Formulation C** |
|---|---|---|---|
| **Ingrédients** | **mg par capsule** | **mg par capsule** | **mg par capsule** |
| Principe actif | 180,60 | 180,60 | 180,60 |
| Triglycérides, chaîne moyenne | 122,85 | 161,10 | 160,20 |
| Graisse dure | 114,75 | 76,50 | 76,50 |
| Lécithine | 1,80 | 1,80 | 2,70 |
| Gélatine | 142,82 | 142,82 | 142,82 |
| Glycérol 85 % | 62,45 | 62,45 | 62,45 |
| Dioxyde de titane | 0,47 | 0,47 | 0,47 |
| Oxyde de fer A | 0,08 | 0,08 | 0,08 |
| Oxyde de fer B | 0,22 | 0,22 | 0,22 |
| **Poids total de la capsule** | **626,04** | **626,04** | **626,04** |

10. Forme posologique pharmaceutique selon la revendication 4 sous la forme d'une capsule de gélatine molle contenant 200 mg d'équivalent de base libre de principe actif, sélectionné parmi les compositions A, B et C suivantes :
| | **Formulation A** | **Formulation B** | **Formulation C** |
|---|---|---|---|
| **Ingrédients** | **mg par capsule** | **mg par capsule** | **mg par capsule** |
| Principe actif | 240,80 | 240,80 | 240,80 |
| Triglycérides, chaîne moyenne | 163,30 | 214,80 | 216,00 |
| Graisse dure | 153,50 | 102,00 | 102,00 |
| Lécithine | 2,40 | 2,40 | 1,20 |
| Gélatine | 203,19 | 203,19 | 203,19 |
| Glycérol 85 % | 102,61 | 102,61 | 102,61 |
| Dioxyde de titane | 0,57 | 0,57 | 0,57 |
| Oxyde de fer A | 0,90 | 0,90 | 0,90 |
| Oxyde de fer B | 0,90 | 0,90 | 0,90 |
| Poids total de la capsule | 868,17 | 868,17 | 868,17 |
